# FASCICULE DE BREVET EUROPEEN

(11) **EP 1 574 202 B1**
(45) Date de publication et mention de la délivrance du brevet: **30.09.2015**
(21) Numéro de dépôt: 05011677.1
(22) Date de dépôt: 31.10.2001
(51) Int. Cl.: A61Q 5/06

(54) **Mousse coiffante longue tenue**
Langhaltender Haarschaum
Hair mousse with long-lasting effect

(30) Priorité: 07.11.2000 FR 0014232
(43) Date de publication de la demande: 14.09.2005
(62) Demande divisionnaire de: 01402832.8
(73) Titulaire: L'Oréal, 75008 Paris (FR)
(72) Inventeur: Belli, Emmanuelle, 92600 Asnières (FR)
(74) Mandataire: Casalonga, Axel

(56) Documents cités:
- EP-A- 1 192 932
- WO-A-00/39176
- WO-A-00/40628
- FR-A- 2 772 605

## Description

La présente invention concerne une mousse de coiffage contenant un type particulier de copolymère acrylique à blocs ramifié, conditionnée dans un dispositif aérosol.

Bien que l'on connaisse dans le domaine du coiffage un très grand nombre de polymères fixants, la plupart d'entre eux présentent un pouvoir fixant limité dans le temps et qui résiste mal à l'humidité.

On a découvert récemment les propriétés de coiffage très intéressantes d'un groupe particulier de copolymères acryliques séquencés (ou à blocs) ramifiés décrits plus en détail ci-dessous.

Ces copolymères, utilisés dans des compositions de coiffage, présentent une combinaison de propriétés physicochimiques et cosmétiques qui en font d'excellents polymères fixants. Ainsi, ces copolymères séquencés s'étalent facilement sur les cheveux, présentent une bonne adhésion aux fibres capillaires, donnent un toucher peu collant, s'éliminent facilement au shampooing et donnent une fixation satisfaisante d'une bonne élasticité, stable dans le temps et qui résiste particulièrement bien à l'humidité.

La demanderesse a découvert que ces polymères se prêtaient parfaitement bien à un conditionnement sous forme de mousse coiffante dans des dispositifs aérosol.

En effet, les mousses de coiffage, qui constituent une formulation généralement très appréciée par les utilisateurs, présentent l'inconvénient de présenter une tenue insuffisante dans le temps.

Or, les nouveaux polymères fixants acryliques à blocs indiqués ci-dessus - lorsqu'ils sont conditionnés sous forme de composition aérosol-donnent des mousses coiffantes ayant des propriétés de fixation satisfaisantes et présentant une très bonne tenue dans le temps. En outre, les propriétés cosmétiques sont d'un bon niveau.

Dans un mode de réalisation, la présente invention, concerne une composition sous forme de mousse de coiffage, conditionnée dans un dispositif aérosol, comprenant
- une phase liquide contenant, dans un milieu liquide cosmétiquement acceptable, (a) au moins un polymère filmogène fixant choisi parmi les copolymères à blocs ramifiés comprenant, comme monomères principaux, au moins un acrylate d'alkyle en C₁₋₂₀ et/ou au moins un N-mono- ou N,N-di-(alkyle en C₂₋₁₂)(méth)acrylamide, et de l'acide acrylique et/ou de l'acide méthacrylique, et au moins un agent tensioactif cationique, anionique ou zwitterionique ou un mélange de ceux-ci, et
- au moins un agent propulseur.

Le polymère filmogène fixant (a) utilisé dans les compositions cosmétiques de la présente invention est un copolymère séquencé (ou à blocs) ramifié ayant une structure constituée de blocs hydrophobes sur lesquels sont fixées, notamment par l'intermédiaire de motifs bifonctionnels, un certain nombre de blocs plus hydrophiles. Ces copolymères présentent au moins deux températures de transition vitreuse.

Ils sont notamment décrits dans la demande de brevet WO 00/40628.

Les copolymères séquencés ramifiés décrits ci-dessus sont proposés par exemple sous les dénominations EX-SDR-26^{®} et EX-SDR-45^{®} par la société GOODRICH.

Ces copolymères présentent la composition suivante :
- de 26 à 36 % en moles d'acide acrylique
- de 27,5 à 30,5 % en moles d'acrylate de *n*-butyle
- de 33,3 à 45,3 % en moles d'acide métacrylique
- de 0,48 à 0,92 % en moles de méthacrylate d'allyle

Les blocs les plus hydrophobes ont un poids moléculaire de 10 000 à 100 000 et les blocs les plus hydrophiles ont un poids moléculaire de 1000 à 100 000 daltons.

Les polymères filmogènes fixants ci-dessus sont utilisés de préférence sous forme anionique, c'est-à-dire sous forme de sel résultant de la neutralisation partielle ou totale des groupes acide (méth)acrylique. L'agent de neutralisation peut être n'importe qu'elle base minérale ou organique physiologiquement acceptable qui n'interfère pas de manière désavantageuse avec le système épaississant. On peut citer à titre d'exemple d'agent de neutralisation préféré le 2-amino-2-méthyl-1-propanol ou l'hydroxyde de sodium.

Le milieu cosmétiquement acceptable est de préférence un milieu aqueux ou hydroalcoolique, et en particulier un milieu aqueux, contenant le ou les polymères fixants séquencés ramifiés à l'état dissous.

La phase liquide contient de préférence entre 0,1 et 10 % en poids, et en particulier entre 0,5 et 5 % en poids de polymère fixant séquencé ramifié, rapporté au poids total de la phase liquide.

Les mousses de coiffage conditionnées dans un dispositif aérosol de la présente invention contiennent un ou plusieurs agents tensioactifs. Ces agents tensioactifs favorisent la formation de mousses fines ayant une certaine stabilité nécessaire à la bonne répartition sur les cheveux.

Les agents tensioactifs peuvent être des agents tensioactifs cationiques, anioniques ou zwitterioniques.

A titre d'exemples d'agents tensioactifs anioniques, on peut citer notamment les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les alkyl-sulfates, les alkyléther-sulfates, les alkylamidoéthersulfates, les alkylarylpolyéthersulfates, les monoglycéride-sulfates, les alkylsulfonates, les phosphates d'alkyle, les alkylamidesulfonates, les alkylarylsulfonates, les α-oléfine-sulfonates, les paraffine-sulfonates, les sulfosuccinates d'alkyle, les alkyléthersulfosuccinates, les alkylamidesulfosuccinates, les sulfoacétates d'alkyle, les acylsarcosinates, et les acylglutamates, les groupes alkyle ou acyle de tous ces composés comportant de 6 à 24 atomes de carbone et le groupe aryle désignant de préférence un groupe phényle ou benzyle.

On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les sulfosuccinamates d'alkyle, les iséthionates d'acyle et les N-acyltaurates, le groupe alkyle ou acyle de tous ces composés comportant de 12 à 20 atomes de carbone. Parmi les tensioactifs anioniques encore utilisables, on peut également citer les lactylates d'acyle dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Les agents tensioactifs amphotères peuvent être notamment des dérivés d'amines aliphatiques secondaires ou tertiaires dans lesquels le groupe aliphatique est une chaîne linéaire ou ramifiée comportant de 8 à 22 atomes de carbone et contenant au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amido-alkyl(C₆-C₈)sulfobétaïnes, et leurs mélanges.

Parmi les dérivés d'amines, on peut citer les produits commercialisés sous la dénomination MIRANOL^{®}, tels que ceux décrits dans les brevets US 2,528,378 et US 2,781,354 et classés dans le dictionnaire CTFA, 3ème édition, 1982, sous les dénominations Amphocarboxyglycinate et Amphocarboxypropionate correspondant respectivement aux formules (a) et (b) :

R₂-CONHCH₂CH₂-N⁺(R₃)(R₄)(CH₂COO⁻) (a)

dans laquelle :
R₂ représente un groupe alkyle dérivé d'un acide R₂-COOH présent dans l'huile de coprah hydrolysée, un groupe heptyle, nonyle ou undécyle,
R₃ représente un groupe bêta-hydroxyéthyle, et
R₄ représente un groupe carboxyméthyle ;
et

R₂'-CONHCH₂CH₂-N(B)(C) (b)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
R₂' représente un groupe alkyle d'un acide R₂'-COOH présent dans l'huile de coprah ou dans l'huile de lin hydrolysée, un groupe alkyle, notamment en C₁₇ et sa forme iso, ou un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL^{®} C2M concentré par la société RHODIA.

La concentration de ces agents tensioactifs dans les mousses aérosol de la présente invention est de préférence comprise entre 0,1 et 10 % en poids, et en particulier entre 0,1 et 4 % en poids, rapporté au poids total de la phase liquide.

On peut conditionner les mousses de coiffage de la présente invention dans des dispositifs aérosols en présence de n'importe quel propulseur usuellement employé pour la préparation de compositions aérosols. On utilisera de préférence des agents propulseurs non solubles ou partiellement solubles dans la phase liquide tels que le diméthyléther, les alcanes enC₃₋₅, le 1,1-difluoroéthane, les mélanges de diméthyléther et d'alcanes en C₃₋₅, et les mélanges de 1,1-difluoroéthane et de diméthyléther et/ou d'alcanes en C₃₋₅.

On préfère tout particulièrement utiliser comme agent propulseur pour les mousses aérosol de la présente invention les alcanes en C₃₋₅, et en particulier le propane, le n-butane et l'isobutane.

Le rapport en poids de la phase liquide à l'agent propulseur des mousses aérosol de la présente invention est de préférence compris entre 70/30 et 98/2 et en particulier entre 85/15 et 96/4.

L'invention a également pour objet un procédé de coiffage consistant à appliquer sur les cheveux une quantité appropriée de la composition de coiffage selon l'invention, à répartir la composition sur les cheveux jusqu'à disparition de la mousse et à sécher ou laisser sécher les cheveux après leur avoir donné la forme désirée.

Les exemples de formulation illustrent la présente invention sans cependant la limiter.

### Exemple

### Mousses coiffantes aérosol

| | **1(réf)** | **3** |
|---|---|---|
| **Ingrédients de la phase liquide (en % en poids)** | | |
| EX-SDR-26^{®a)} | 2 | 1 |
| Celquat^{®} LOR^{d)} | - | 0,5 |
| DC 2 1388^{®e)} | - | 5 |
| Tégo-bétaïne^{®} HS^{f)} | - | 0,5 |
| Brij^{®} 30^{g)} | - | 0,5 |
| Ethanol | 8,2 | - |
| Eau | q.s.p. 100 g | q.s.p. 100 g |
| **rapport phase liquide/ propulseur^{h)}** | 95/5 | 95/5 |

| | | |
|---|---|---|
| ^{a)}copolymère à blocs ramifié commercialisé par la société GOODRICH ^{d)} copolymère d'hydroxyéthylcellulose et de chlorure de diallyldiméthylammonium commercialisé par la société NATIONAL STARCH. ^{e)}polydiméthylsiloxane-α,ω-dihydroxyle(10)/cyclopentadiméthylsiloxane (90) en émulsion aqueuse à 60 % commercialisé par la société DOW CORNING ^{f)}mélange cocoylamidopropylbétaïne(25)/monolaurate de glycéryle(5) à 30 % dans l'eau commercialisé par la société GOLDSCHMIDT ^{g)}alcool laurylique éthoxylé par 4 moles d'oxyde d'éthylène commercialisé par la société UNIQEMA ^{h)}mélange isobutane(56)/butane(24)/propane(20) commercialisé sous la dénomination AEROGAZ^{®} 3.2 N par la société ATOCHEM | | |

## Revendications

1. Composition sous forme de mousse de coiffage, conditionnée dans un dispositif aérosol, comprenant
- une phase liquide contenant, dans un milieu liquide cosmétiquement acceptable, (a) au moins un polymère filmogène fixant choisi parmi les copolymères à blocs ramifiés comprenant, comme monomères principaux, au moins un acrylate d'alkyle en C₁₋₂₀ et/ou au moins un N-mono- ou N,N-di-(alkyle en C₂₋₁₂)(méth)acrylamide, et de l'acide acrylique et/ou de l'acide méthacrylique, et au moins un agent tensioactif cationique, anionique ou zwitterionique ou un mélange de ceux-ci, et
- au moins un agent propulseur.

2. Composition de coiffage selon la revendication 1, **caractérisée par le fait que** le polymère filmogène fixant (a) est un copolymère à blocs ramifié comprenant, comme monomères, de l'acrylate de n-butyle, de l'acide acrylique, de l'acide méthacrylique et du méthacrylate d'allyle.

3. Composition de coiffage selon la revendication 2, **caractérisée par le fait que** le polymère filmogène fixant (a) est constitué de 26 à 36 % en moles d'acide acrylique, de 27,5 à 30,5 % en moles d'acrylate de n-butyle, de 33,3 à 45,3 % en moles d'acide méthacrylique et de 0,48 à 0,92 % en moles de méthacrylate d'allyle

4. Composition de coiffage selon l'une quelconque des revendications 1 à 3, **caractérisée par le fait que** le milieu cosmétiquement acceptable est un milieu aqueux ou hydroalcoolique et de préférence un milieu aqueux.

5. Composition de coiffage selon l'une des revendications 1 à 4, **caractérisée par le fait que** la concentration du polymère filmogène fixant (a) est comprise entre 0,1 et 10 % en poids rapporté au poids total de la phase liquide.

6. Composition de coiffage selon la revendication 5, **caractérisée par le fait que** la concentration du polymère filmogène fixant (a) est comprise entre 0,5 et 5 % en poids, rapporté au poids total de la phase liquide.

7. Composition de coiffage selon l'une des revendications 1 à 6, **caractérisée par le fait que** la concentration du ou des agent(s) tensioactifs est comprise entre 0,1 et 10 % en poids, et de préférence entre 0,1 et 4 % en poids, rapporté au poids total de la phase liquide.

8. Composition de coiffage selon l'une quelconque des revendications 1 à 7, **caractérisée par le fait que** la phase liquide contient en outre (b) un ou plusieurs polymères filmogènes, différents du polymère filmogène fixant (a).

9. Composition de coiffage selon la revendication 8, **caractérisée par le fait que** la concentration du ou des polymère(s) filmogène(s) fixant(s) (b) est comprise entre 0,1 et 10 % en poids, de préférence entre 0,1 et 5 % en poids rapporté au poids total de la phase liquide.

10. Composition de coiffage selon l'une quelconque des revendications 1 à 9, **caractérisée par le fait que** l'agent propulseur est choisi parmi le diméthyléther, les alcanes en C₃₋₅, le 1,1-difluoroéthane, les mélanges de diméthyléther et d'alcanes en C₃₋₅, et les mélanges de 1,1-difluoroéthane et de diméthyléther et/ou d'alcanes en C₃₋₅.

11. Composition de coiffage selon la revendication 10, **caractérisée par le fait que** l'agent propulseur est choisi parmi les alcanes en C₃₋₅.

12. Composition de coiffage selon l'une quelconque des revendications 1 à 11, **caractérisée par le fait que** le rapport en poids de la phase liquide à l'agent propulseur est compris entre 70/30 et 98/2, de préférence entre 85/15 et 96/4.

13. Procédé de coiffage consistant à appliquer sur les cheveux une quantité appropriée de composition de coiffage selon l'une quelconque des revendications précédentes, à répartir la composition sur les cheveux jusqu'à disparition de la mousse et à sécher ou laisser sécher les cheveux après leur avoir donné la forme désirée.

## Patentansprüche

1. Zusammensetzung in Form von Haarfestigerschaum, wobei sie in einer Aerosolvorrichtung verpackt ist und Folgendes umfasst
- eine Flüssigphase, die in einem kosmetisch unbedenklichen flüssigen Milieu, (a) mindestens ein festigendes filmbildendes Polymer, das aus den verzweigten Blockcopolymeren ausgewählt ist, welche als Hauptmonomere mindestens ein C₁₋₂₀-Alkylacrylat und/oder mindestens ein N-Mono- oder N,N-Di-(C₂₋₁₂-Alkyl)(meth)acrylamid sowie Acrylsäure und/oder Methacrylsäure umfassen, sowie mindestens ein kationisches, anionisches oder zwitterionisches Tensid oder eine Mischung daraus enthält, und
- mindestens ein Treibmittel.

2. Haarfestigerzusammensetzung nach Anspruch 1, **dadurch gekennzeichnet, dass** das festigende filmbildende Polymer (a) ein verzweigtes Blockcopolymer ist, welches als Monomere n-Butylacrylat, Acrylsäure, Methacrylsäure und Allylmethacrylsäure umfasst.

3. Haarfestigerzusammensetzung nach Anspruch 2, **dadurch gekennzeichnet, dass** das festigende filmbildende Polymer (a) zu 26 bis 36 mol-% aus Acrylsäure, zu 27,5 bis 30,5 mol-% aus n-Butylacrylat, zu 33,3 bis 45,3 mol-% aus Methacrylsäure und zu 0,48 bis 0,92 mol-% aus Allylmethacrylat besteht.

4. Haarfestigerzusammensetzung nach einem beliebigen der Ansprüche 1 bis 3, **dadurch gekennzeichnet, dass** es sich bei dem kosmetisch unbedenklichen Milieu um ein wässriges oder wässrig-alkoholisches Milieu handelt, und vorzugsweise um ein wässriges Milieu.

5. Haarfestigerzusammensetzung nach einem der Ansprüche 1 bis 4, **dadurch gekennzeichnet, dass** die Konzentration des festigenden filmbildenden Polymers (a) im Bereich von 0,1 bis 10 Gewichts-% liegt, bezogen auf das Gesamtgewicht der Flüssigphase.

6. Haarfestigerzusammensetzung nach Anspruch 5, **dadurch gekennzeichnet, dass** die Konzentration des festigenden filmbildenden Polymers (a) im Bereich von 0,5 bis 5 Gewichts-% liegt, bezogen auf das Gesamtgewicht der Flüssigphase.

7. Haarfestigerzusammensetzung nach einem der Ansprüche 1 bis 6, **dadurch gekennzeichnet, dass** die Konzentration des oder der Tenside im Bereich von 0,1 bis 10 Gewichts-% liegt, und vorzugsweise von 0,1 bis 4 Gewichts-%, bezogen auf das Gesamtgewicht der Flüssigphase.

8. Haarfestigerzusammensetzung nach einem beliebigen der Ansprüche 1 bis 7, **dadurch gekennzeichnet, dass** die Flüssigphase darüber hinaus (b) ein oder mehrere filmbildende Polymere enthält, die sich von dem festigenden filmbildenden Polymer (a) unterscheiden.

9. Haarfestigerzusammensetzung nach Anspruch 8, **dadurch gekennzeichnet, dass** die Konzentration des oder der festigenden filmbildenden Polymers/Polymere (b) im Bereich von 0,1 bis 10 Gewichts-% liegt, und vorzugsweise von 0,1 bis 5 Gewichts-%, bezogen auf das Gesamtgewicht der Flüssigphase.

10. Haarfestigerzusammensetzung nach einem beliebigen der Ansprüche 1 bis 9, **dadurch gekennzeichnet, dass** das Treibmittel aus Dimethylether, C₃₋₅-Alkanen, 1,1-Difluorethan, den Mischungen aus Dimethylether und C₃₋₅-Alkanen und den Mischungen aus 1,1-Difluorethan und Dimethylether und/oder C₃₋₅-Alkanen ausgewählt ist.

11. Haarfestigerzusammensetzung nach Anspruch 10, **dadurch gekennzeichnet, dass** das Treibmittel aus den C₃₋₅-Alkanen ausgewählt ist.

12. Haarfestigerzusammensetzung nach einem beliebigen der Ansprüche 1 bis 11, **dadurch gekennzeichnet, dass** das Gewichtsverhältnis der Flüssigphase zum Treibmittel im Bereich von 70/30 bis 98/2, vorzugsweise von 85/15 bis 96/4, liegt.

13. Haarfestigungsverfahren, das darin besteht, eine geeignete Menge der Haarfestigerzusammensetzung nach einem beliebigen der vorhergehenden Ansprüche auf das Haar aufzubringen, die Zusammensetzung auf dem Haar zu verteilen, bis der Schaum nicht mehr wahrnehmbar ist, und das Haar zu trocknen oder trocknen zu lassen, nachdem ihm die gewünschte Form verliehen wurde.

## Claims

1. Composition in the form of a styling mousse, packaged in an aerosol device, comprising
- a liquid phase comprising, in a cosmetically acceptable liquid medium, (a) at least one fixing film-forming polymer chosen from branched block copolymers comprising, as main monomers, at least one C₁₋₂₀ alkyl acrylate and/or at least one N-mono-or N,N-di(C₂₋₁₂ alkyl) (meth) acrylamide, and acrylic acid and/or methacrylic acid, and at least one cationic, anionic or zwitterionic surface-active agent or a mixture of these, and
- at least one propellant.

2. Styling composition according to Claim 1, **characterized in that** the fixing film-forming polymer (a) is a branched block copolymer comprising, as monomers, n-butyl acrylate, acrylic acid, methacrylic acid and allyl methacrylate.

3. Styling composition according to Claim 2, **characterized in that** the fixing film-forming polymer (a) is composed of 26 to 36 mol% of acrylic acid, of 27.5 to 30.5 mol% of n-butyl acrylate, of 33.3 to 45.3 mol% of methacrylic acid and of 0.48 to 0.92 mol% of allyl methacrylate.

4. Styling composition according to any one of Claims 1 to 3, **characterized in that** the cosmetically acceptable medium is an aqueous or aqueous/alcoholic medium and preferably an aqueous medium.

5. Styling composition according to one of Claims 1 to 4, **characterized in that** the concentration of the fixing film-forming polymer (a) is between 0.1 and 10% by weight with respect to the total weight of the liquid phase.

6. Styling composition according to Claim 5, **characterized in that** the concentration of the fixing film-forming polymer (a) is between 0.5 and 5% by weight with respect to the total weight of the liquid phase.

7. Styling composition according to one of Claims 1 to 6, **characterized in that** the concentration of the surface-active agent(s) is between 0.1 and 10% by weight, and preferably between 0.1 and 4% by weight, with respect to the total weight of the liquid phase.

8. Styling composition according to any one of Claims 1 to 7, **characterized in that** the liquid phase additionally comprises (b) one or more film-forming polymers other than the fixing film-forming polymer (a).

9. Styling composition according to Claim 8, **characterized in that** the concentration of the fixing film-forming polymer(s) (b) is between 0.1 and 10% by weight, preferably between 0.1 and 5% by weight, with respect to the total weight of the liquid phase.

10. Styling composition according to any one of Claims 1 to 9, **characterized in that** the propellant is chosen from dimethyl ether, C₃₋₅ alkanes, 1,1-difluoroethane, mixtures of dimethyl ether and of C₃₋₅ alkanes, and mixtures of 1,1-difluoroethane and of dimethyl ether and/or of C₃₋₅ alkanes.

11. Styling composition according to Claim 10, **characterized in that** the propellant is chosen from C₃₋₅ alkanes.

12. Styling composition according to any one of Claims 1 to 11, **characterized in that** the ratio by weight of the liquid phase to the propellant is between 70/30 and 98/2, preferably between 85/15 and 96/4.

13. Styling process which consists in applying, to the hair, an appropriate amount of the styling composition according to any one of the preceding claims, in spreading the composition over the hair until the mousse has disappeared and in then drying the hair or allowing it to dry after it has been given the desired form.
